(19) Europäisches Patentamt
European Patent Office
Office européen des brevets

(11) Publication number: 0 120 403
B1

(12) EUROPEAN PATENT SPECIFICATION

(45) Date of publication of patent specification: 10.06.87

(51) Int. Cl.$^4$: G 03 C 1/02, G 03 C 5/54, C 07 C 125/00, G 03 C 1/06

(21) Application number: 84102744.4

(22) Date of filing: 13.03.84

(54) **Heat developable color photographic materials.**

(30) Priority: 16.03.83 JP 43861/83

(43) Date of publication of application: 03.10.84 Bulletin 84/40

(45) Publication of the grant of the patent: 10.06.87 Bulletin 87/24

(84) Designated Contracting States: DE FR GB NL

(56) References cited:
EP-A-0 066 282
US-A-3 420 665

CHEMICAL ABSTRACTS, vol. 66, 1967, page 1022, no. 10690w, Columbus, Ohio, US; N. SHARGHI et al.: "o-Trifluoromethylthiophenol and its derivatives"

(73) Proprietor: FUJI PHOTO FILM CO., LTD.
210 Nakanuma Minami Ashigara-shi
Kanagawa 250-01 (JP)

(72) Inventor: Hirai, Hiroyuki c/o Fuji Photo Film Co., Ltd.
No. 210, Nakanuma
Minami Ashigara-shi Kanagawa (JP)
Inventor: Sato, Kozo c/o Fuji Photo Film Co., Ltd.
No. 210, Nakanuma
Minami Ashigara-shi Kanagawa (JP)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22 (DE)

EP 0 120 403 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a heat developable color photographic material comprising a support having thereon at least a photosensitive silver halide, a binder, a dye-releasing material which is reductive to the photosensitive silver halide and releases a hydrophilic mobile dye upon reaction with the photo-sensitive silver halide by heating, and of base precursor.

The "base precursor" in this invention is a material releasing a basic component by thermal decomposition.

Since the photographic process using silver halide has excellent photographic properties such as sensitivity and gradation control as compared with other photographic processes, such as, for example, an electrophotographic process and a diazo photographic process, the silver halide photographic process has been most widely used. Recently, however, a technique of easily and rapidly obtaining images by employing a dry process such as heating, as the image-forming process of a photographic material using silver halide in place of a conventional wet processing using developing solution, has been developed.

Heat developable photographic materials are known in the art and heat developable photographic materials and processes for processing these photographic materials are described in, for example, *Shashin Kogaku no Kiso* (The Basis of Photographic Engineering), pages 553—555, published by Corona K.K., 1979; *Eizo Joho* (Image Information), page 40, published April, 1978; *Nebletts Handbook of Photography and Reprography,* 7th Ed., pages 32—33, published by Van Nostrand Reinhold Company; U.S. Patent Nos. 3,152,904; 3,301,678; 3,392,020; 3,457,075; U.K. Patent Nos. 1,131,108; 1,167,777; and *Research Disclosure* June 1978, pages 9—15 (RD-17029).

Various processes of obtaining color images have been proposed. For example, a process for forming color images by the combination of the oxidation product of a developing agent and couplers involving p-phenylenediamine reducing agents and phenolic or active methylene couplers are described in U.S. Patent No. 3,531,286; p-aminophenol reducing agents are described in U.S. Patent No. 3,761,270; sulfonamidophenol reducing agents are described in Belgian Patent No. 802,519 and Research Disclosure, September 1975, pages 31 and 32; and the combination of sulfonamidophenol reducing agents and 4-equivalent couplers are described in U.S. Patent No. 4,021,240.

However, these processes have the disadvantage that turbid color images are formed since reduced silver images and the colour images are simultaneously formed at the unexposed areas after heat development. To overcome this disadvantage, a process of removing the silver images by liquid treatment and a process of transferrring the dye only onto another layer, for example, a sheet having an image-receiving layer have been proposed, however, it is not easy to discriminate the dye from unreacted materials and transfer only the dye.

Also, a process of introducing a nitrogen-containing heterocyclic group into a dye, forming a silver salt, and liberating the dye by heat development in the presence of the silver salt is described in *Research Disclosure,* May 1978, pages 54—58 (RD-16966). However, in this process, clear images cannot be obtained since it is difficult to control the liberation of dye at the unexposed areas and hence the process is unsuitable for general use.

Furthermore, a process of forming positive color images using heat-sensitive silver dye bleaching process is known with useful dyes and bleaching processes being described in, for example, *Research Disclosure,* April 1976, pages 30—32 (RD-14433), *ibid.,* December 1976, pages 14—15 (RD-15227); and U.S. Patent No. 4,235,957.

However, the foregoing processes have the disadvantages that an additional step of superposing an activating agent sheet followed by heating is required for accelerating the bleaching of the dye and also the color image formed is gradually bleached by reduction due to free silver which is present during storage of the color image material for a long period of time.

Also, a process of producing color images utilizing leuco dyes is described in, for example, U.S. Patent Nos. 3,985,565 and 4,022,617. However, the process had the disadvantage that it is difficult to retain leuco dyes in photographic materials in a stable manner and hence the photographic material gradually becomes colored during storage.

EP—A—066282 discloses a heat developable color photographic material comprising a support having thereon a photosensitive silver halide, a binder, a dye-releasing material which is reductive to the photosensitive halide and releases a hydrophilic mobile dye upon reaction with the photosensitive silver halide by heating and a base precursor. Said base precursor is preferably a salt of a carboxylic acid and an organic base. Examples of suitable carboxylic acids include trichloroacetic acid and trifluroacetic acid.

It is the object of this invention to provide a heat developable color photographic material capable of providing a high-density color image having less fog in a short period of time and having excellent stability with the passage of time. By the term "stability with the passage of time" is meant that the change in photographic properties such as the maximum density, the minimum density or the sensitivity, is less during the storage of the photographic material before heat development.

Said objects are achieved by a heat developable color photographic material comprising a support having thereon at least a photosensitive silver halide, a binder, a dye-releasing material which is reductive to the photosensitive silver halide and releases a hydrophilic dye upon reaction with the photosensitive

silver halide by heating, and a base precursor, characterized in that the base precursor is represented by the general formula I

$$\{R\!-\!(SO_2CH_2CO_2H)_x\}_z\cdot B_y \qquad (I)$$

wherein

R represents an alkyl group, an alkylene group, an aryl group , an arylene group, a monovalent or divalent heterocyclic group, each of which may be unsubstituted or substituted;

B represents a monoacidic or diacidic base having a pKa of not lower than 9 and containing 12 carbon atoms or less;

x is 1 when R represents a monovalent group or 2 when R represents a divalent group;

y is the same as x when B represents a monoacidic base or when B represents a diacidic base; and

z is 2 when R represents a monovalent group and B represents a diacidic base or otherwise 1.

R may be an alkyl group having 1 to 22 carbon atoms, an alkylene group having 1 to 22 carbon atoms, an aryl group having 6 to 22 carbon atoms, an arylene group having 6 to 22 carbon atoms, or a monovalent or divalent 5-or ·6-membered heterocyclic group containing N, S and/or O as hetero atom(s) or its condensed ring group, each of which may be unsubstituted or substituted.

Preferred condensed heterocyclic groups include the above heterocyclic groups condensed with a benzene ring.

Preferred examples of R include an aryl group, an arylene group, a monovalent or divalent heterocylic group, and substituted counterparts of these groups. Particularly preferred examples of R include aryl groups or heterocyclic groups (including condensed heterocyclic groups) substituted with an electron attracting group having a Hammet sigma value of above 0 (for example, a halogen atom, a cyano group, a nitro group, a carbamoyl group or a sulfamoyl group).

Of the bases represented by B, bases with low volatility and having a pKa value of not lower than 10 and a boiling point of not lower than 150°C are preferred. Most preferred examples of bases for B include guanidines, cyclic guanidines, amidines, cyclic amidines and tetraalkylammonium hydroxide.

Preferred specific examples of R include a methylene group, an ethylene group, a phenyl group, a p-chlorophenyl group, a p-bromophenyl group, a p-iodophenyl group, a 3,4-dichlorophenyl group, a 2,5-dichlorophenyl group, a 2,4-dichlorophenyl group, a 3-nitrophenyl group, a 4-nitrophenyl group, a 4-cyanophenyl group, a 3-carbamoylphenyl group, a 3-sulfamoylphenyl group, a 2-methoxycarbonylphenyl group, a 3-methoxycarbonylphenyl group, a 1-naphthyl group, a 2-naphthyl group, a 2-pyridyl group, a 4-pyridyl group, a 2-thienyl group, a 5-chloro-2-thienyl group, a 2-benzimidazolyl group, a 1,3-phenylene group, a 1,5-naphthylene group, a 2,6-naphthylene group and a 2,7-naphthylene group.

Preferred specific examples of bases represented by B include dimethylamine, diethylamine, piperidine, piperazine, ethylenediamine, N,N'-dimethylethylenediamine, acetoamine, diazabicyclononene, diazabicycloundecene, tetramethylammonium hydroxide, tetraethylammonium hydroxide,

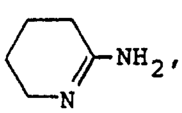, 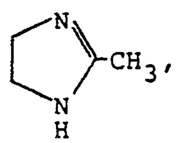, 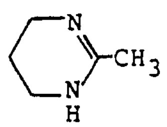,

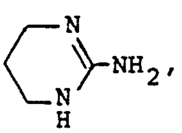, 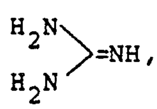, 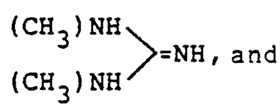, and 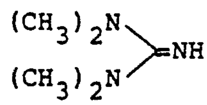.

The following are preferred examples of the base precursor which can be used in the present invention.

(1)

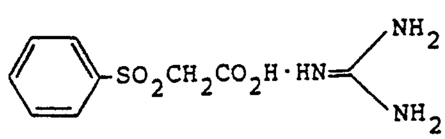

(2)

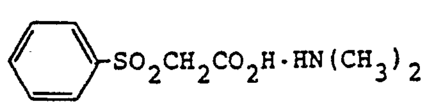

(3)

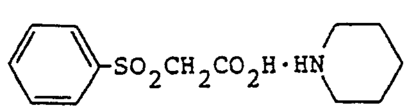

(4)

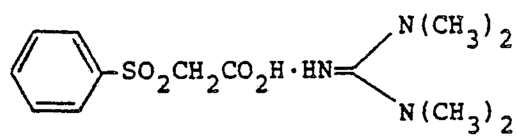

(5)

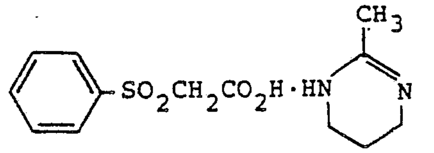

(6)

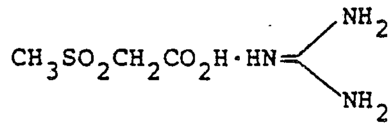

(7)

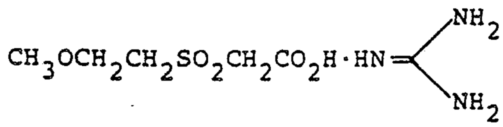

(8)

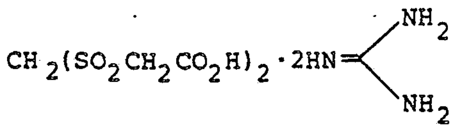

(9)

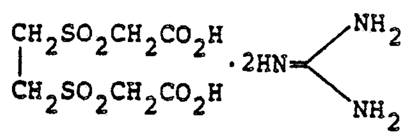

(10)

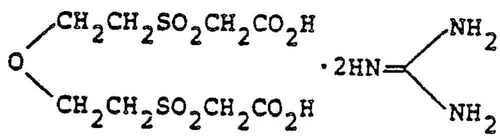

(11)

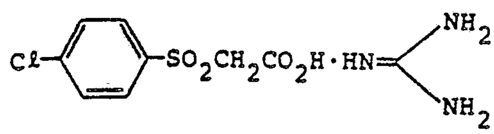

(12)

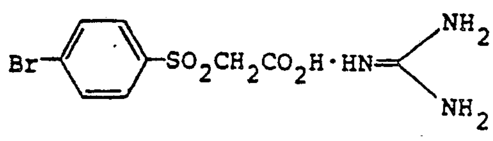

(13)

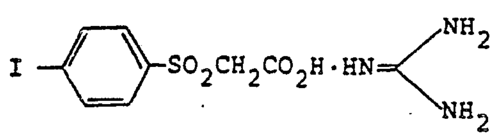

(14)

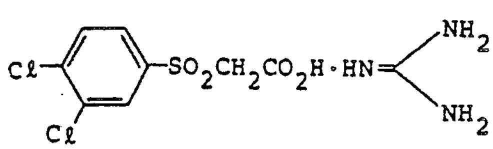

(15)

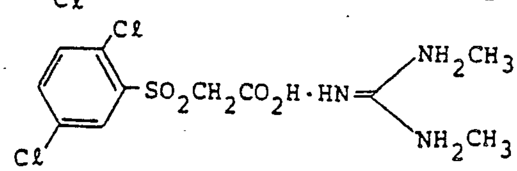

(16)

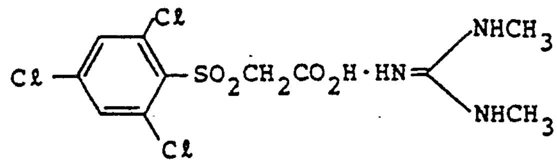

(17)

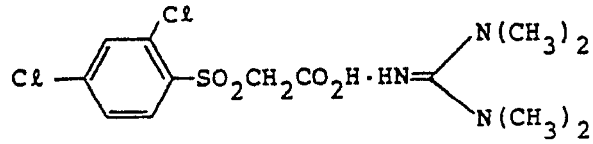

(18)

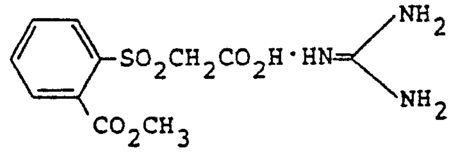

(19)

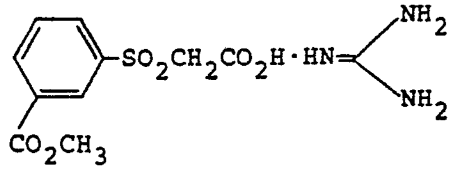

(20)

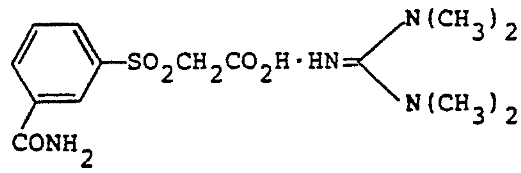

(21)

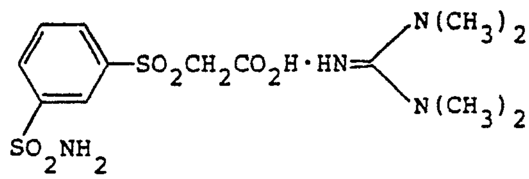

(22)

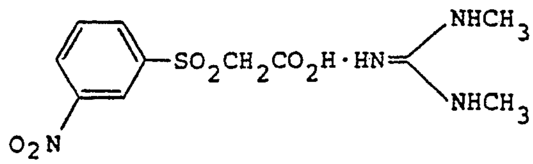

(23)

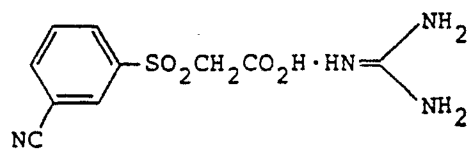

(24)

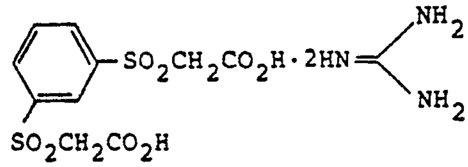

(25)

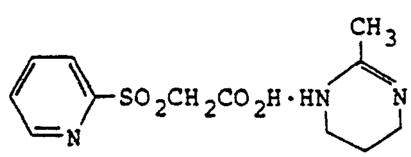

(26)

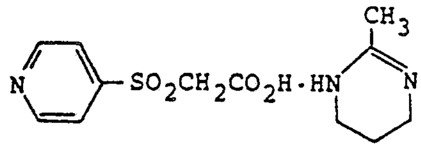

(27)

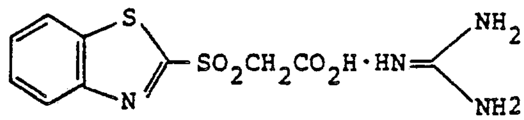

(28)

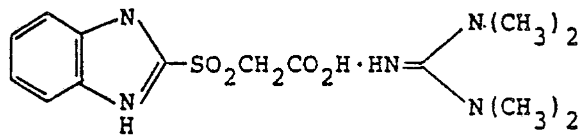

(29)

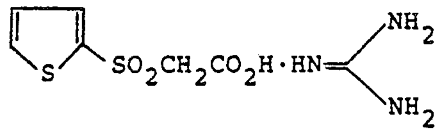

(30)

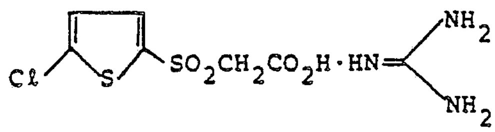

(31)

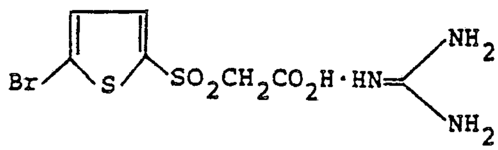

(32)

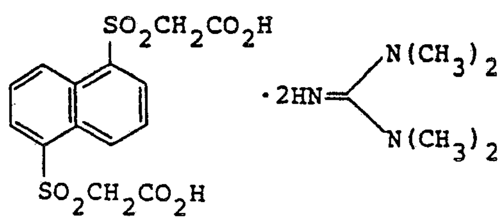

(33)

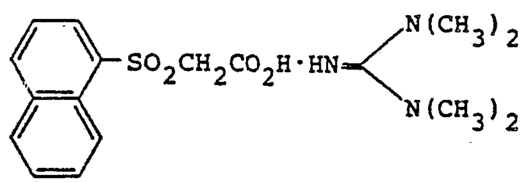

(34)

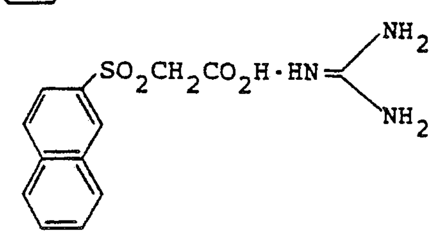

(35)

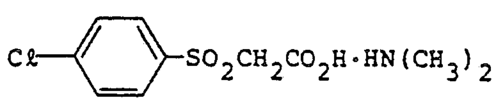

(36)

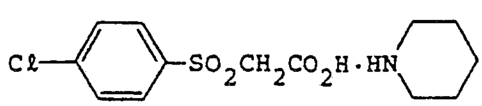

(37)

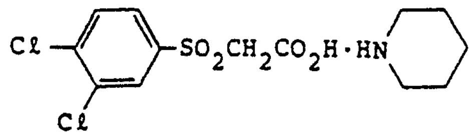

(38)

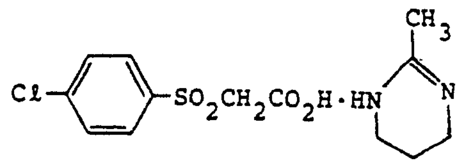

(39)

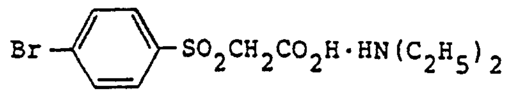

(40)

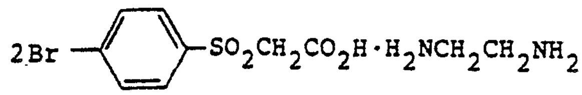

(41)

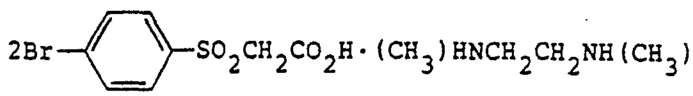

(42)

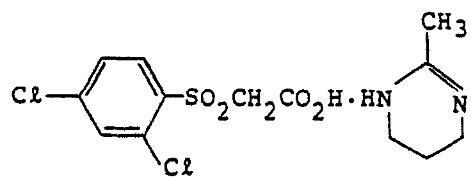

(43)

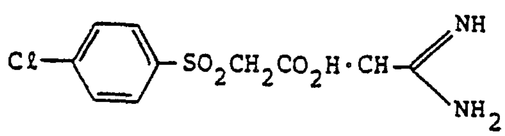

(44)

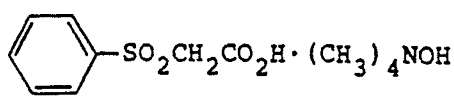

(45)

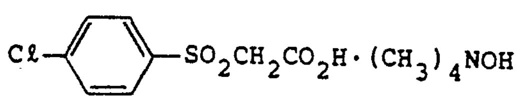

(46)

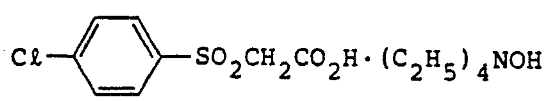

The α-sulfonylacetate represented by the general formula I can generally be prepared by condensing a sulfinic acid with an α-haloacetic acid ester to form an α-sulfonylacetic acid ester, subjecting this ester to hydrolysis with an alkali at room temperature to obtain an α-sulfonylacetic acid and converting the acid to its salt in a conventional method.

The synthesis of examples of the base precursors of the present invention are shown below. Unless otherwise indicated, all parts percents, ratios and the like are by weight.

Synthesis Example 1

Synthesis of Base Precursor (1)

A mixture of 60 g of sodium benzenesulfinate dihydrate, 33.4 g of ethyl bromoacetate and 300 ml of methanol was heated under reflux for 2 hours. After the methanol was distilled off under reduced pressure, water and ethyl acetate were added and the organic layer formed was separated. The organic layer was washed with water and dried followed by distilling off the ethyl acetate under reduced pressure to obtain 43.3 g of ethyl phenylsulfonyl acetate as a pale yellow liquid.

The ester thus obtained was added to a 15% potassium hydroxide aqueous solution and the mixture was stirred at room temperature for 1 hour. While cooling with ice, the mixture was neutralized with diluted hydrochloric acid and extracted with ethyl acetate. The organic layer was separated, washed with water and dried. Distilling off the ethyl acetate under reduced pressure gave rise to 28 g of phenylsulfonylacetic acid as colorless crystals.

A mixture of 20 g of phenylsulfonylacetic acid, 9 g of guanidine carbonate and 100 ml of methanol was stirred at room temperature for 1 hour. Distilling off methanol (at a temperature of 50°C or less) afforded 25.5 g of Base Precursor (1). Melting point (decomp): 137—138°C.

Synthesis Example 2

Synthesis of Base Precursor (11)

To a mixture of 504 g of sodium sulfite and 1.8 l of water was added portionwise 16 g of p-chlorobenzenesulfonyl chloride at 50°C. After being stirred at 55—60°C for 3 hours, the mixture was cooled with ice and white crystals which separated out were collected and washed with cold water to obtain 152 g of sodium p-chlorobenzensulfinate. In a manner similar to Synthesis Example 1, sodium p-chlorobenzenesulfinate and ethyl chloroacetate were reacted to obtain ethyl p-chlorophenylsulfonyl acetate. The ester thus obtained (110 g) was added portionwise to a 20% sodium hydroxide aqueous solution while cooling with ice. During this procedure, white crystals separated out quickly. After stirring at room temperature for 1 hour, the mixture was cooled with ice and white crystals were collected by filtration and washed with isopropanol. The crystals thus obtained were dissolved in 300 ml of water and the solution was neutralized with diluted hydrochloric acid. The white crystals which separated out were collected by filtration, washed with water to obtain 90 g of p-chlorophenylsulfonylacetic acid.

A mixture of 82 g of p-chlorophenylsulfonylacetic acid, 31.5 g of guanidine carbonate, 200 ml of methanol and 100 ml of water was stirred at room temperature for 3 hours. The white crystals which separated out were collected by filtration and washed with methanol to obtain 88 g of Base Precursor (11). Melting point (decomp.): 147—148°C.

The base precursor used in this invention can be employed in a wide range of amounts. For example, it is advantageous for the base precursor to be used in a coverage of less than 50% by weight, preferably 0.01 to 40% by weight based on the dry weight of the coating.

The base precursor in this invention may be used alone or as a mixture of two or more of the base precursors or they may be used in combination with other known base precursor or precursors.

The base precursors in this invention can be incorporated in the binder as a solution in a water miscible organic solvent (for example, methanol, ethanol, acetone or dimethyl formamide) or in a mixture of such an organic solvent and water. The base precursor in this invention can be prepared by admixing an acid ($R(SO_2CH_2CO_2H)$) and an organic base (B) in the presence of a suitable solvent and a suitable vehicle (e.g., a coating solution) and can be used as it is.

The heat developable color photographic material of this invention simultaneously provides a silver image and a mobile dye or mobile dyes at the area corresponding to the silver image supply by heat development of the color photographic material after image-wise exposure.

The term "heat development" as used herein means heating a heat developable color photographic material, after image-wise exposure or simultaneously with image-wise exposure, in a substantially water-free state so as to provide an exposed silver halide and a dye-releasing material, thereby releasing a dye.

When the heat developable color photographic material of this invention is heated after being image-wise exposed or simultaneously with image-wise exposure in a substantially water-free state, an oxidation reduction reaction occurs between the photosensitive silver halide and the reducing dye-releasing material with the exposed photosensitive silver halide to form a silver image at the exposed area. In this step the dye-releasing material is oxidized by the exposed photosensitive silver halide to form the oxidation product thereof, whereby a hydrophilic mobile dye is released and a silver image and the mobile dye are obtained at the exposed area. In this case, when a base exists in the system, the above-described reaction is accelerated. By transferring the mobile dye or dyes onto a dye-fixing layer, a dye image is obtained. However, if a base is directly incorporated in a color photographic material, the stability of the photographic material with the passage of time is reduced. On the other hand, in the case of using the base precursor of this invention, a base is released when developing the photographic material containing the base precursor by heating at high temperature. Accordingly, the stability of the photographic material with the passage of time can be improved.

The foregoing explanation relates to using a negative-type silver halide emulsion. An explanation using an autopositive silver halide emulsion is the same except that the silver image and the mobile dye are obtained in the unexposed area.

A feature of this invention is that the oxidation reduction reaction of the photosensitive silver halide and the dye-releasing material and the dye releasing reaction subsequently occur at high temperature in a substantially water free state. The term "high temperature" as used herein is a temperature higher than 80°C and the term "substantially water free state" is a state which is in an equilibrium relation with the moisture in the air but water is not supplied to the system. Such a state is described in *The Theory of the Photographic Process,* 4th Ed., page 374, Edited by T. H. James, published by Macmillan Co.

The system of this invention exhibits sufficient reactivity even in the substantially water free state and

this can be confirmed from the fact that when a sample is dried in vacuum of $10^{-3}$ mmHg for one day, the reactivity thereof is not reduced.

Hitherto, it has been believed that a dye-releasing reaction occurs due to an attack of a so-called nucleophilic reagent and the dye-releasing reaction is ususlly performed in a liquid with a pH of not lower than 10. Therefore, it is unexpected that the heat developable color photographic material of this invention shows a high reactivity under high temperature in a substantially water free dry state. Also, the dye-releasing material of this invention can result in an oxidation reduction reaction with silver halide without requiring the assistance of a so-called auxiliary developing agent. This is also unexpected from conventional knowledge about wet development at temperatures near normal temperature.

When an organic silver salt oxidizing agent is present in the reaction system, the foregoing reaction proceeds well and results in a high image density. Accordingly, a particularly preferred embodiment in this invention involves an organic silver salt oxidizing agent in the reaction system.

The dye releasing redox compound which releases a hydrophilic diffusible dye which can be used in the present invention is a compound described in EP—A—76,492, as a dye releasing compound and is represented by the following general formula:

$$R_a—SO_2—D$$

wherein $R_a$ represents a reducing group capable of begin oxidized by the silver halide; and D represents an image forming dye moiety containing a hydrophilic group.

The above-described compound is oxidized corresponding to or in a reverse manner corresponding to the latent image distributed imagewise in the silver halide and a mobile dye is released in an imagewise manner.

Detailed definitions of $R_a$ and D, examples of the specific compounds and synthesis examples thereof are described in EP—A—76,492.

Suitable dye releasing redox compounds which can also be used in the present invention include the compounds as described, for example, in U.S. Patent 4,055,428, Japanese Patent Application (OPI) Nos. 12642/81, 16130/81, 16131/81, 650/82 and 4043/82, U.S. Patents 3,928,312 and 4,076,529, U.S. Published Patent Application B 351,673, U.S. Patents 4,135,929 and 4,198,235, Japanese Patent Application (OPI) No. 46730/78, U.S. Patents 4,273,855, 4,149,892, 4,142,891 and 4,258,120.

These compounds are also effective in addition to the above-described compounds.

Further, the dye releasing redox compounds which release a yellow dye as described, for example, in U.S. Patents 4,013,633, 4,156,609, 4,148,641, 4,165,987, 4,148,643, 4,183,755, 4,246,414, 4,268,625 and 4,245,023, Japanese Patent Application (OPI) Nos. 71072/81, 25737/81, 138744/80, 134849/80, 106727/77, and 114930/76 can be effectively used in the present invention.

The dye releasing redox compounds which release a magenta dye as described, for example, in U.S. Patents 3,954,476, 3,932,380, 3,931,144, 3,932,381, 4,268,624 and 4,255,509, Japanese Patent Application (OPI) Nos. 73057/81, 71060/81, 134850/80, 40402/80, 36804/80, 23628/78, 106727/77, 33142/80 and 53329/80 can be effectively used in the present invention.

The dye releasing redox compounds which release a cyan dye as described, for example, in U.S. Patents 3,929,760, 4,013,635, 3,942,987, 4,273,708, 4,148,642, 4,183,754, 4,147,544, 4,165,238, 4,246,414 and 4,268,625, Japanese Patent Application (OPI) Nos. 71061/81, 47823/78, 8827/77 and 143323/78 can be effectively used in the present invention.

Two or more of the dye releasing redox compounds can be used together, if desired. In these cases, two or more dye releasing redox compounds may be used together in order to achieve the same hue or in order to achieve a black color.

The dye releasing redox compounds are suitably used in a range from about 10 $mg/m^2$ to about 15 $g/m^2$ and preferably in a range from 20 $mg/m^2$ to 10 $g/m^2$ in a total.

The dye releasing redox compound of the formula I used in the present invention can be introduced into a layer of the light-sensitive material using known methods such as a method as described in U.S. Patent 2,322,027. In this case, an organic solvent having a high boiling point or an organic solvent having a low boiling point as described below can be used. For example, the dye releasing redox compound can be dispersed in a hydrophilic colloid after it is dissolved in an organic solvent having a high boiling point, for example, a phthalic acid alkyl ester (for example, dibutyl phthalate or dioctyl phthalate), a phosphoric acid ester (for example, diphenyl phosphate, triphenyl phosphate, tricresyl phosphate, or dioctylbutyl phosphate), a citric acid ester (for example, tributyl acetylcitrate), a benzoic acid ester (for example, octyl benzoate), an alkylamide (for example, diethyl laurylamide), an aliphatic acid ester (for example, dibutoxyethyl succinate or dioctyl azelate), a trimesic acid ester (for example, tributyl trimesate), or an organic solvent having a low boiling point of about 30° to 160°C, for example, a lower alkyl acetate such as ethyl acetate, or butyl acetate, ethyl priopionate, secondary butyl alcohol, methyl isobutyl ketone, β-ethoxyethyl acetate, methyl cellosolve acetate or cyclohexanone. The above-described organic solvents having a high boiling point and organic solvents having a low boiling point may be used as a mixture thereof, if desired.

Further, it is possible to use a dispersion method employing a polymer as descrbed in Japanese Patent Publication No. 39853/76 and Japanese Patent Application (OPI) No. 59943/76. Moreover, various surface

active agents can be used when the dye releasing redox compound is dispersed in a hydrophilic colloid. For this purpose, the surface active agents illustrated hereinafter in the specification can be used.

In the present invention, if desired, a reducing agent may also be used. The reducing agent in this case is a so-called auxiliary developing agent, which is oxidized by the silver halide and/or the organic silver salt oxidizing agent to form an oxidized product having the ability to oxidize the reducing group Y in the dye releasing redox compound of the general formula I.

Examples of useful auxiliary developing agents include the compounds specifically described in EP—A No. 76,492.

Suitable reducing agents which can be used in the present invention include the following compounds: hydroquinone compounds (for example, hydroquinone, 2,5-dichlorohydroquinone or 2-chlorohydroquinone), aminophenol compounds (for example, 4-aminophenol, N-methylaminophenol, 3-methyl-4-aminophenol, or 3,5-dibromoaminophenol), catechol compounds (for example, catechol, 4-cyclohexylcatechol, 3-methoxycatechol, or 4-(N-octadecylamino)catechol), phenylenediamine compounds (for example, N,N-diethyl-p-phenylenediamine, 3-methyl-N,N-diethyl-p-phenylenediamine, 3-methoxy-N-ethyl-N-ethoxy-p-phenylenediamine, or N,N,N',N'-tetramethyl-p-phenylenediamine).

Various combinations of developing agents as described in U.S. Patent 3,039,869 can also be used.

In the present invention, the amount of the reducing agent which can be employed is from 0.01 mol to 20 mols per mol of silver and more preferably from 0.1 mol to 10 mols per mol of silver.

The silver halide used in the present invention can be silver chloride, silver chlorobromide, silver chloroiodide, silver bromide, silver iodobromide, silver chloroiodobromide and silver iodide.

In the embodiment of the present invention in which the organic silver salt oxidizing agent is not used together with but the silver halide is used alone, a particularly preferred silver halide is silver halide partially containing a silver iodide crystal in the particles. That is, a silver halide in which the X-ray diffraction pattern shows that of pure silver iodide is particularly preferred.

In photographic materials a silver halide containing two or more kinds of halogen atoms can be used. Such a silver halide yields a completely mixed crystal in a conventional silver halide emulsion. For example, the particles of silver iodobromide show an X-ray diffraction pattern at a position corresponding to the mixed ratio of silver iodide crystal and silver bromide crystal but not at a position corresponding to pure silver iodide crystal and pure silver bromide crystal separately.

Particularly preferred examples of silver halides which can be used in the present invention include silver chloroiodide, silver iodobromide, and silver chloroiodobromide each containing silver iodide crystals in the particles thereof and showing X-ray diffraction pattern of silver iodide crystals.

The process for preparing those silver halides using silver iodobromide is exemplary. That is, silver iodobromide is prepared by first adding a silver nitrate solution to a potassium bromide solution to form silver bromide particles and then adding potassium iodide to the mixture.

Two or more kinds of silver halides in which the particle size and/or a halogen composition are different from each other may be used in admixture, if desired.

The average particle size of the silver halide used in the present invention is preferably from about 0.001 μm to about 10 μm and more preferably from 0.001 μm to 5 μm.

The silver halide used in the present invention may be used as it is. However, the silver halide may also be chemically sensitized with a chemical sensitizing agent such as compounds of sulfur, selenium or tellurium, or compounds of gold, platinum, palladium, rhodium or iridium, a reducing agent such as tin halide, or a combination thereof. The details thereof are described in T. H. James, *The Theory of the Photographic Process,* the Fourth Edition, Chapter 5, pages 149 to 169.

In a particularly preferred embodiment of the present invention, an organic silver salt oxidizing agent is also used. The organic silver salt oxidizing agent is a silver salt which forms a silver image upon reaction with the above-described image forming substance or a reducing agent which are copresent, if desired, with the image forming substance, when it is heated to a temperature of above about 80°C and, preferably, above 100°C in the presence of exposed silver halide. Due to the copresence of the organic silver salt oxidizing agent, a light-sensitive material which provides higher color density can be obtained.

The silver halide used in this case does not always need to have the characteristic that the silver halide contains pure silver iodide crystals in the case of using the silver halide alone. Any silver halide which is known in the art can be used.

Examples of such organic silver salt oxidizing agents include those described in European Patent Application (OPI) No. 76,492.

A silver salt of an organic compound having a carboxy group can be used. Typical examples thereof include a silver salt of an aliphatic carboxylic acid and a silver salt of an aromatic carboxylic acid.

In addition, a silver salt of a compound containing a mercapto group or a thione group and a derivative thereof can be used.

Further, a silver salt of a compound containing an imino group can be employed. Suitable examples of these compounds include a silver salt of benzotriazole and the derivatives thereof as described in Japanese Patent Publication Nos. 30270/69 and 18416/70, for example, a silver salt of benzotriazole, a silver salt of alkyl-substituted benzotriazole such as a silver salt of methylbenzotriazole, a silver salt of a halogen-substituted benzotriazole such as a silver salt of 5-chlorobenzotriazole, a silver salt of carboimidobenzotriazole such as a silver salt of butylcarboimidobenzotriazole, a silver salt of 1,2,4-triazole

or 1-H-tetrazole as described in U.S. Patent 4,220,709, a silver salt of carbazole, a silver salt of saccharin, or a silver salt of imidazole and an imidazole derivative.

Moreover, a silver salt as described in *Research Disclosure,* Vol. 170, No. 17029 (June, 1978) and an organic metal salt such as copper stearate, are organic metal salt oxidizing agents capable of being used in the present invention.

Methods of preparing these silver halide and organic silver salt oxidizing agents and techniques of blending them are described in *Research Disclosure,* No. 17029, Japanese Patent Application (OPI) Nos. 32928/75 and 42529/76, U.S. Patent 3,700,458, and Japanese Patent Application (OPI) Nos. 13224/74 and 17216/75.

A suitable coating amount of the light-sensitive silver halide and the organic silver salt oxidizing agent employed in the present invention is in a total of from 50 mg/m$^2$ to 10 g/m$^2$ calculated as silver.

The light-sensitive silver halide and the organic silver salt oxidizing agent used in the present invention are prepared in the binder as described below. Further, the dye releasing redox compound used in the present invention is dispersed in the binder described below.

The binder which can be used in the present invention can be employed alone or as a combination thereof. A hydrophilic binder can be used as the binder according to the present invention. Typical hydrophilic binders are transparent or translucent hydrophilic colloids, examples of which include natural substances, for example, a protein such as gelatin, a gelatin derivative or a cellulose derivative, a polysaccharide such as starch or gum arabic, and a synthetic polymer, for example, a water-soluble polyvinyl compound such as polyvinyl alcohol, polyvinyl pyrrolidone or acrylamide polymer. Another example of a synthetic polymer compound is a dispersed vinyl compound in a latex form which is used for the purpose of increasing the dimensional stability of the photographic material.

The silver halide used in the present invention can be spectrally sensitized with methine dyes or other dyes. Suitable dyes which can be employed include cyanine dyes, merocyanine dyes, complex cyanine dyes, complex merocyanine dyes, holopolar cyanine dyes, hemicyanine dyes, styryl dyes, and hemioxonol dyes. Of these dyes, cyanine dyes, merocyanine dyes and complex merocyanine dyes are particularly useful. Any conventionally utilized nucleus for cyanine dyes, such as basic heterocyclic nuclei, can be employed in these dyes. That is, a pyrroline nucleus, an oxazoline nucleus, a thiazoline nucleus, a pyrrole nucleus, an oxazole nucleus, a thiazole nucleus, a selenazole nucleus, an imidazole nucleus, a tetrazole nucleus or a pyridine nucleus, and further, nuclei formed by condensing alicyclic hydrocarbon rings with these nuclei and nuclei formed by condensing aromatic hydrocarbon rings with these nuclei, that is, an indolenine nucleus, a benzindolenine nucleus, an indole nucleus, a benzoxazole nucleus, a naphthoxazole nucleus, a benzothiazole nucleus, a naphthothiazole nucleus, a benzoselenazole nucleus, a benzimidazole nucleus or a quinoline nucleus, are appropriate. The carbon atoms of these nuclei may also be substituted, if desired.

Suitable merocyanine dyes and complex merocyanine dyes are those having nuclei with a ketomethylene structures 5- or 6-membered heterocyclic nuclei such as a pyrazolin-5-one nucleus, a thiohydantoin nucleus, a 2-thiooxazolidin-2, 4-dione nucleus, a thiazolidin-2,4-dione nucleus, a rhodanine nucleus or a thiobarbituric acid nucleus.

These sensitizing dyes can be employed alone, and can also be employed as a combination thereof. A combination of sensitizing dyes is often used, particularly for the purpose of supersensitization. Representative examples thereof are described in U.S. Patents 2,688,545, 2,977,229, 3,397,060, 3,522,052, 3,527,641, 3,617,293, 3,628,964, 3,666,480, 3,672,898, 3,679,428, 3,703,377, 3,769,301, 3,814,609, 3,837,862 and 4,026,707, British Patents 1,344,281 and 1,507,803, Japanese Patent Publication Nos. 4936/68 and 12375/78, and Japanese Patent Application (OPI) Nos. 110618/77 and 109925/77.

The sensitizing dyes may be present in the emulsion together with dyes which themselves do not provide spectrally sensitizing effects but exhibit a supersensitizing effect or materials which do not substantially absorb visible light but exhibit a supersensitizing effect. For example, amino-stilbene compounds substituted with a nitrogen-containing heterocyclic group (e.g., those described in U.S. Patents 2,933,390 and 3,635,721), aromatic organic acid-formaldehyde condensates (e.g., those described in U.S. Patent 3,743,510), cadmium salts or azaindene compounds, can be employed. The combinations described in U.S. Patents 3,615,613, 3,615,641, 3,617,295 and 3,635,712 are particularly useful.

Suitable supports used in the light-sensitive material and the dye fixing material employed, if desired, according to the present invention are supports which can endure the processing temperature. As ordinary support, such as glass, paper, metal or analogues thereof may be used, but also an acetyl cellulose film, a cellulose ester film, a polyvinyl acetal film, a polystyrene film, a polycarbonate film, a polyethylene terephthalate film, and a film related thereto or a synthetic resin material may be used. Further, a paper support laminated with a polymer such as polyethylene can be used. The polyesters described in U.S. Patents 3,634,089 and 3,725,070 are preferably used.

In the present invention, various kinds of dye releasing activators can be used. The term "dye releasing activator" refers to a substance which can accelerate the oxidation-reduction reaction of the light-sensitive silver halide and/or the organic silver salt oxidizing agent with a dye releasing redox compound or which, in the subsequent dye releasing reaction, can accelerate the release of a dye as a result of its nucleophilic attack to the oxidized dye releasing redox compound. A base and a base precursor can be used as the dye

releasing activator. It is particularly advantageous to use dye releasing activators in order to accelerate the reactions in the present invention.

Examples of preferred bases are amines which include trialkylamines, hydroxylamines, aliphatic polyamines, N-alkyl substituted aromatic amines, N-hydroxyalkyl substituted aromatic amines and bis[p-(dialkylamino)phenyl]methanes. Further, betaine tetramethylammonium iodide and diaminobutane dihydrochloride as described in U.S. Patent 2,410,644, and urea and organic compounds including amino acids such as 6-aminocaproic acid as described in U.S. Patent 3,506,444 can be used.

It is advantageous to use a compound represented by the general formula described below in the heat-developable color photographic material in order to accelerate development and accelerate release of a dye.

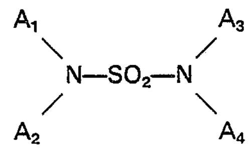

wherein $A_1$, $A_2$, $A_3$ and $A_4$, which may be the same or different, each represents a hydrogen atom or a substituent selected from the group consisting of an alkyl group, a substituted alkyl group, a cycloalkyl group, an aralkyl group, an aryl group, substituted aryl group and a heterocyclic group; and $A_1$ and $A_2$ or $A_3$ and $A_4$ may combine with each other to form a ring.

The above-described compound can be used in a broad range of amounts. A useful range is up to about 20% by weight based on the weight of a dry layer coated of the light-sensitive material. A range of 0.1% by weight to 15% by weight is more preferred.

It is advantageous to use a water releasing compound in the present invention in order to accelerate the dye releasing reaction.

The water releasing compound means a compound which releases water upon decomposition during heat development. These compounds are particularly known in the field of printing of fabrics, and for example $NH_4Fe(SO_4)_2 \cdot 12H_2O$, as described in Japanese Patent Application (OPI) No. 88386/75 is useful.

Further, in the present invention, it is possible to use a compound which activates development simultaneously while stabilizing the image. Particularly, isothiuroniums including 2-hydroxyethyliso-thiuronium trichloroacetate as described in U.S. Patent 3,301,678 bisisothiuroniums including 1,8-(3,6-di-oxaoctane)-bis(isothiuronium trifluoroacetate), as described in U.S. Patent 3,669,670, thiol compounds as described in German Patent Application (OLS) No. 2,162,714, thiazolium compounds such as 2-amino-2-thiazolium trichloroacetate, or 2-amino-5-bromoetyl-2-thiazolium trichloroacetate, as described in U.S. Patent 4,012,260, compounds having α-sulfonylacetate as an acid moiety such as bis(2-amino-2-thiazolium)-methylenebis(sulfonylacetate) or 2-amino-2-thiazolium phenylsulfonylacetate, as described in U.S. Patent 4,060,420, and compounds having 2-carboxycarboxamide as an acid part as described in U.S. Patent 4,088,496 are preferred for use.

In the present invention, it is possible to use a thermal solvent. The term "thermal solvent" means a non-hydrolyzable organic material which is solid at ambient temperature but melts together with the other components at a temperature of the heat treatment or below. Preferred examples of thermal solvents include compounds acting as a solvent for the developing agent and compounds having a high dielectric constant which accelerate physical development of silver salts. Examples of preferred thermal solvents include those described in European Patent Application (OPI) No. 76,492.

In the present invention, though it is not very necessary to further incorporate substances or dyes for preventing irradiation or halation in the light-sensitive material, because the light-sensitive material is colored by the dye releasing redox compound, it is possible to use filter dyes or light absorbing materials, as described in Japanese Patent Publication No. 3692/73 and U.S. Patents 3,253,921, 2527,583 and 2,956,879, in order to further improve the sharpness. Preferably these dyes have a thermal bleaching property. For example, the dyes as described in U.S. Patents 3,769,019, 3,745,009 and 3,615,432 are preferred.

The light-sensitive material of the present invention may contain, if desired, various additives known for heat-developable light-sensitive materials and may include a layer other than the light-sensitive layer, for example, an antistatic layer, an electrically conductive layer, a protective layer, an intermediate layer, an antihalation layer or a strippable layer.

The photographic emulsion layer and other hydrophilic colloid layers in the light-sensitive material of the present invention may contain various surface active agents for various purposes, for example, as coating aids or for prevention of electrical charging, improvement of lubricating property, emulsification, prevention of adhesion or improvement of photographic properties (for example, acceleration of development, providing a hard tone or sensitization).

For example, nonionic surface active agents such as saponin (steroid), alkylene oxide derivatives (for example, polyethylene glycol, polyethylene glycol/polypropylene glycol condensates, polyethylene glycol alkyl ethers or polyethylene glycol alkylaryl ethers, polyethylene glycol esters, polyethylene glycol sorbitan esters, polyalkylene glycol alkylamine or amides or polyethylene oxide adducts of silicone), glycidol derivatives (for example, alkenylsuccinic acid polyglycerides or alkylphenol polyglycerides), polyhydric

alcohol aliphatic acid esters or saccharide alkyl esters, anionic surface active agents containing acid groups such as a carboxy group, a sulfo group, a phospho group, a sulfate group or a phosphate group, such as alkylcarboxylic acid salts, alkylsulfonic acid salts, alkylbenzenesulfonic acid salts, alkylnaphthalenesulfonic acid salts, alkyl sulfuric acid esters, alkylphosphoric acid esters, N-acyl-N-alkyltaurines, sulfosuccinic acid esters, sulfoalkyl polyoxyethylene alkylphenyl ethers, or polyoxyethylene alkylphosphoric acid esters; ampholytic surface active agents such as amino acids, aminoalkylsulfonic acids, aminoalkylsulfuric acid esters or phosphoric acid esters, alkylbetaines or amine oxides, and cationic surface active agents such as alkylamine salts, aliphatic or aromatic quaternary ammonium salts, heterocyclic quaternary ammonium salts such as pyridinium salts or imidazolium salts, aliphatic or heterocyclic phosphonium salts or aliphatic or heterocyclic solfonium salts, can be used.

Of the above-described surface active agents, polyethylene glycol type nonionic surface active agents having ethylene oxide recurring units in their structure are preferably incorporated into the light-sensitive material. It is particularly preferred for the structure to contain 5 or more of ethylene oxide recurring units.

The nonionic surface active agents capable of satisfying the above-described conditions are well known as to their structures, properties and methods of synthesis. These nonionic surface active agents are widely used even outside this field. Representative references describing these agents include: *Surfactant Science Series,* Vol. 1, Nonionic Surfactants (edited by Martin J. Schick, Marcel Dekker Inc., 1967), and *Surface Active Ethylene Oxide Adducts* (edited by Schoufeldt N. Pergamon Press, 1969). Suitable nonionic surface active agents described in the above-mentioned references, are those capable of satisfying the above-described conditions and they are preferably employed in connection with the present invention.

The nonionic surface active agents can be used alone or as a mixture of two or more thereof, if desired.

Polyethylene glycol type nonionic surface active agents can be used in an amount of less than about 100% by weight, preferably less than 50% by weight, based on the weight of the hydrophilic binder present.

The light-sensitive material of the present invention may contain a cationic compound containing a pyridinium salt. Examples of cationic compounds containing a pyridinium group which can be used are described in *PSA Journal,* Section B 36 (1953), U.S. Patents 2,648,604 and 3,671,247, Japanese Patent Publication Nos. 30074/69 and 9503/69.

The photographic emulsion layer and other binder layers of the photographic light-sensitive material and the dye fixing material of the present invention may contain inorganic or organic hardeners. Chromium salts (chromium alum, or chromium acetate), aldehydes (formaldehyde, glyoxal, or glutaraldehyde), N-methylol compounds (dimethylolurea, methylol or dimethylhydantoin), dioxane derivatives (2,3-dihydroxydioxane, active vinyl compounds (1,3,5-triacryloylhexahydro-s-triazine, or 1,3-vinylsulfonyl-2-propanol), active halogen compounds (2,4-dichloro-6-hydroxy-s-triazine) or mucohalogenic acids, mucochloric acid, or mucophenoxychloric acid), can be used alone or as a combination thereof.

Examples of various additives include those described in *Research Disclosure,* Vol. 170, No. 17029 (June, 1978), for example, plasticizers, dyes for improving sharpness, antihalation dyes, sensitizing dyes, matting agents, fluorescent whitening agents and fading preventing agents.

If desired, two or more layers may be coated at the same time by the method as described in U.S. Patent 2,761,791 and British Patent 837,095.

Various means of exposure can be used in the present invention. Latent images are obtained by imagewise exposure to radiant light including visible rays. Generally, light sources used for conventional color prints can be used, examples of which include tungsten lamps, mercury lamps, halogen lamps such as iodine lamps, xenon lamps, laser light sources, CRT light sources, fluorescent tubes and light-emitting diodes.

In the present invention, simultaneously with or after the heat-developable color photographic material is exposed to light, the resulting latent image can be developed by heating the entire material to a suitably elevated temperature, for example, about 80°C to about 250°C for about 0.5 second to about 300 seconds. A higher temperature or lower temperature can be utilized with heating time, if it is within the above-described temperature range, being prolonged or shortened. Particularly, a temperature range of about 110°C to about 160°C is useful.

A simple heat plate, iron, heat roller or heat generator utilizing carbon or titanium white, or analogues thereof may be used as the heating means.

In the present invention, a specific method for forming a color image by heat development comprises transfer of a hydrophilic mobile dye. For this purpose, the heat-developable color photographic material of the present invention comprises a support having thereon a light-sensitive layer containing at least a silver halide, an organic silver salt oxidizing agent, a dye releasing redox compound which is also a reducing agent for the organic silver salt oxidizing agent and a binder, and a dye fixing layer capable of receiving the hydrophilic diffusible dye formed in the light-sensitive layer.

The above described light-sensitive layer and the dye fixing layer may be formed on the same support, or they may be formed on different supports, respectively. The dye fixing layer can be stripped off the light-sensitive layer. For example after the heat-developable color photographic material is exposed imagewise to light, it is developed by heating uniformly and thereafter the dye fixing layer or the light-sensitive layer is peeled apart. Also, when a light-sensitive material having the light-sensitive layer coated on a support and a fixing material having the dye fixing layer coated on a support are separately formed, after the light-sensitive material is exposed imagewise to light and uniformly heated, the mobile dye can be transferred to the dye fixing layer by superposing the fixing material on the light-sensitive material.

Further, a method wherein only the light-sensitive layer is exposed imagewise to light and heated uniformly by superposing the dye fixing layer light-sensitive layer.

The dye fixing layer can contain, for example, a dye mordant in order to fix the dye. In the present invention, various mordants can be used, and polymer mordants are particularly preferred. In addition to the mordants, the dye fixing layer may contain bases, base precursors and thermal solvents as previously described. In particular, incorporation of the bases or base precursors into the dye fixing layer is particularly preferred where the light-sensitive layer and the dye fixing layer are formed on different supports.

Polymer mordants which can be used in the present invention are polymers containing secondary and tertiary groups, polymers containing nitrogen-containing heterocyclic moieties, polymers with quaternary cation groups thereof, having a molecular weight of from about 5,000 to about 200,000, and particularly from 10,000 to 50,000.

For example, vinylpyridine polymers and vinylpyridinium cation polymers as disclosed in U.S. Patents 2,548,564, 2,484,430, 3,148,061 and 3,756,814, polymer mordants capable of cross-linking with gelatin as disclosed in U.S. Patents 3,625,694, 3,859,096 and 4,128,538 and British Patent 1,277,453, aqueous sol type mordants as disclosed in U.S. Patents 3,958,995, 2,721,852 and 2,798,063, Japanese Patent Application (OPI) Nos. 115228/79, 145529/79 and 126027/79, water-insoluble mordants as disclosed in U.S. Patent 3,898,088, reactive mordants capable of forming covalent bonds with dyes used as disclosed in U.S. Patent 4,168,976 (Japanese Patent Application (OPI) No. 13733/79, and mordants disclosed in U.S. Patents 3,709,690, 3,788,855, 3,642,482, 3,488,706, 3,557,066, 3,271,147 and 3,271,148, Japanese Patent Application (OPI) Nos. 71332/75, 30328/78, 155528/77, 125/78 and 1024/78, are illustrative.

In addition, the mordants disclosed in U.S. Patents 2,675,316 and 2,882,156 can be used.

The dye fixing layer can have a white reflective layer. For example, a layer of titanium dioxide dispersed in gelatin can be provided on the mordant layer on a transparent support. The layer of titanium dioxide forms a white opaque layer, by which reflection color images of the transferred color images which is observed through the transparent support are obtained.

A typical dye fixing material used in the present invention is obtained by mixing a polymer containing ammonium salt groups with gelatin and applying the mixture to a transparent support.

The transfer of dye from the light-sensitive layer to the dye fixing layer can be carried out using a dye transfer assistant. Examples of useful dye transfer assistants include water and an alkaline aqueous solution containing sodium hydroxide, potassium hydroxide and an inorganic alkali metal salt. Further, a solvent having a low boiling point such as methanol, N,N-dimethylformamide, acetone or diisobutyl ketone, and a mixture of such a solvent having a low boiling point with water or an alkaline aqueous solution can be used. The dye transfer assistant can be employed by wetting the image receiving layer with the transfer assistant or by incorporating it in the form of water of crystallization or microcapsules into the material.

The following examples illustrate the invention.

Unless otherwise indicated all parts, ratios and percentages are by weight.

Example 1

In 3,000 ml of water were dissolved 40 g of gelatin and 26 g of potassium bromide and the solution was stirred at 50°C. Then, a solution of 34 g of silver nitrate in 200 ml of water was added to the foregoing solution over a period of 10 minutes and thereafter a solution of 3.3 g of potassium iodide in 100 ml of water was added thereto over a period of 2 minutes.

The pH of the silver iodobromide emulsion was controlled to precipitate excessive salts, which were removed. Thereafter, the pH of the emulsion was adjusted to 6.0 to provide 400 g of a silver iodobromide emulsion.

Then, a dispersion of a dye-releasing material was prepared as follows.

To 30 ml of ethyl acetate were added 5 g of Dye-Releasing Material (1) shown below, 0.5 g of a surface active agent, succinic acid-2-ethyl-hexyl ester sodium sulfonate, and 5 g of tricesyl phosphate and the mixture was heated to about 60°C to form a solution. The solution was mixed with 100 g of a 10% gelatin aqueous solution with stirring and the resultant mixture was treated in a homogenizer at 10,000 r.p.m. for 10 minutes to form a dispersion of a dye-releasing material.

Dye Releasing Material (1)

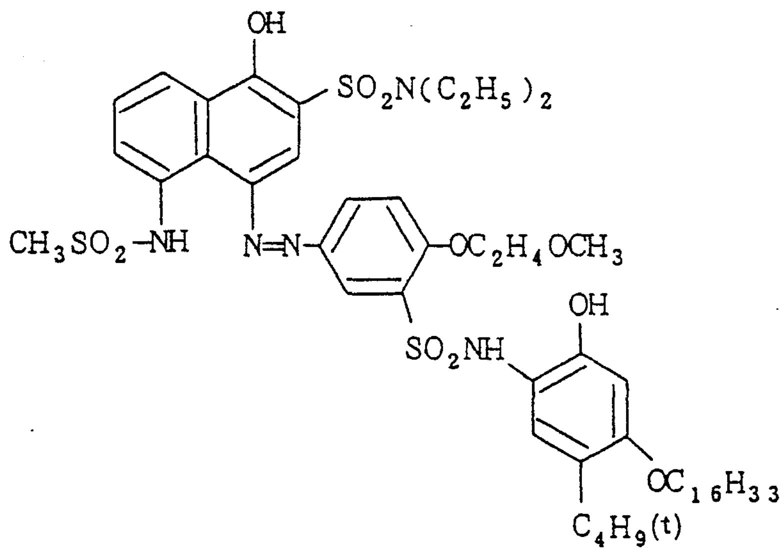

Then, a light-sensitive material was prepared as follows.

| | | |
|---|---|---|
| (a) | Photosensitive Silver Iodobromide Emulsion (as described above) | 25 g |
| (b) | Dispersion of Dye-Releasing Material (as described above) | 33 g |
| (c) | 5% Aqueous Solution of the Following Compound | 10 ml |
| | 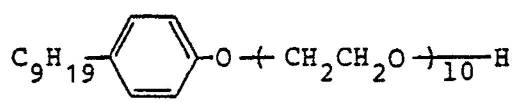 | |
| (d) | 10% Aqueous Solution of the Following Compound | 4 ml |
| | $H_2NSO_2N(CH_3)_2$ | |
| (e) | Solution of 3 g of Base Precursor (1) of this invention in 30 ml of 50:50 vol % water-methanol mixture | |

A mixture of the foregoing components (a) to (e) was heated to form a solution and the solution was coated on a polyethylene terephthalate film of a thickness of 180 μm at a wet thickness of 30 μm to provide a light-sensitive coated material.

After drying, the coated sample was imagewise exposed to light from tungsten lamp at 2,000 lux for 10 seconds and thereafter, the sample was uniformly heated on a heat block heated to 150°C for 30 seconds to provide Sample A.

Then, by following the same procedure as used in producing Sample A except that 30 ml of 50:50 vol % water-methanol was used in place of component (e) above containing Base Precursor (1) of this invention, Sample B was prepared.

An image-receiving material having an image-receiving layer was prepared as follows.

In 200 ml of water was dissolved 10 g of polymethyl acrylate-co-N,N,N-trimethyl-N-vinylbenzylammonium chloride) (the weight ratio of methyl acrylate and vinylbenzylammonium chloride was 1:1) and the solution was uniformly mixed with 100 g of a 10% aqueous solution of lime-treated gelatin. The mixture was uniformly coated on a paper support having laminated thereon a layer of polyethylene with titanium dioxide dispersed therein at a wet thickness of 90 μm and dried to provide an image-receiving material.

After wetting the image-receiving material with water, each of the foregoing heated light-sensitive materials, Samples A and B, was superposed on the image-receiving material so that the coated layers were in a face-to-face relationship.

After heating the assembly on a heat block heated to 80°C for 6 seconds, the image-receiving material was separated from the light-sensitive material, a negative magenta dye image was obtained on the image-receiving material. When the density of the negative image was measured using a Macbeth reflection densitometer (RD—519), the following results were obtained.

| Sample No. | Maximum Density | Minimum Density |
|---|---|---|
| A (This Invention) | 1.95 | 0.20 |
| B (Comparison) | 0.03 | 0.03 |

From the above results, it can be seen that the use of the base precursor of this invention results in high density.

Furthermore, when Sample A was stored for 2 days at 60°C and then processed in the same manner as described above, the minimum density and the maximum density were 0.28 and 1.99 respectively, which indicates that the sample of this invention also has excellent shelf life.

Example 2

By following the same procedure as described in Example 1 except that the base precursors shown in the table below were used in the amounts shown in the same table, the following results were obtained.

| Sample No. | Base Precursor | Maximum Density |
|---|---|---|
| C | Compound (2) 3.0 g | 1.72 |
| D | Compound (3) 3.0 g | 1.80 |
| E | Compound (4) 3.0 g | 1.92 |
| F | Compound (5) 3.0 g | 1.89 |
| G | Compound (6) 3.0 g | 1.70 |
| H | Compound (7) 3.0 g | 1.77 |
| I | Compound (24) 3.0 g | 1.93 |

From the above results, it can be seen that the base precursors of this invention have excellent effects.

Example 3

By following the same procedure as described in Example 1 except that each of the following dye-releasing materials was used in place of the Dye-Releasing Material (1) in Example 1, the following dispersions of dye-releasing materials were prepared.

| | | |
|---|---|---|
| Dye-Releasing Material (2) | 5 g | Dispersion (I) |
| Dye-Releasing Material (3) | 7.5 g | Dispersion (II) |
| Dye-Releasing Material (4) | 5 g | Dispersion (III) |

Dye-Releasing Material (2)

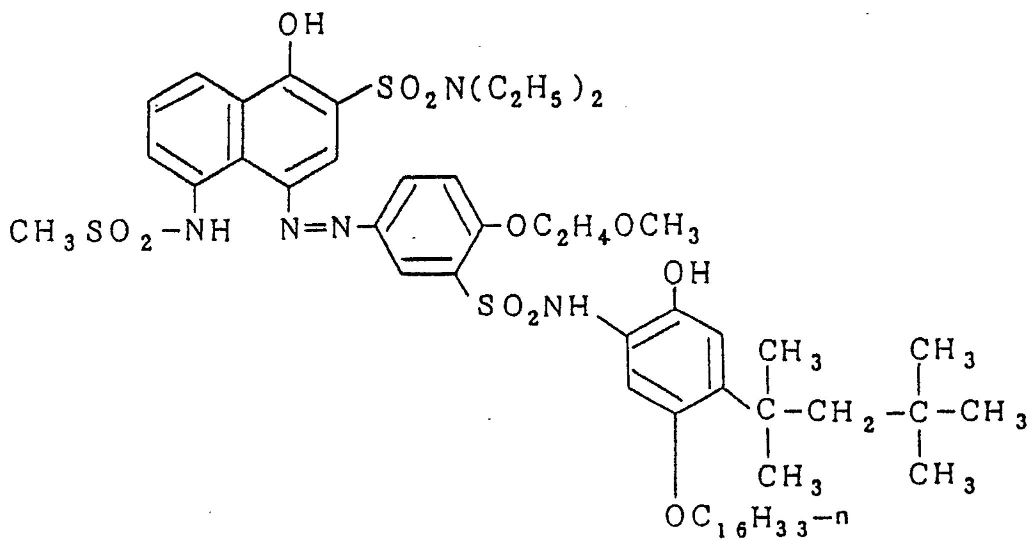

Dye-Releasing Material (3)

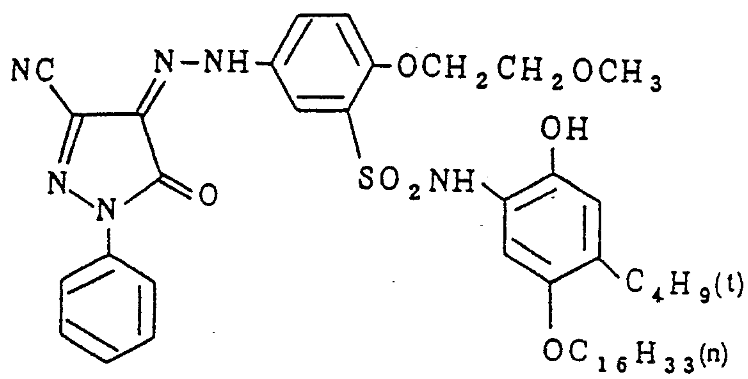

Dye-Releasing Material (4)

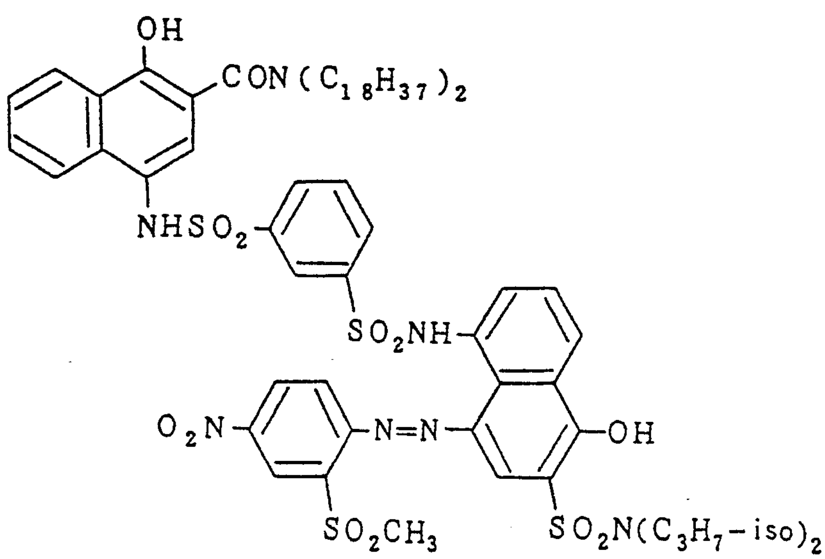

Also, by following the same procedure as described in Example 1, light-sensitive samples were prepared and they were processed as described in Example 1. The results obtained are shown in the following table.

| Dispersion of Dye-Releasing Material | Compound (1) of this Invention | Maximum Density | Minimum Density |
|---|---|---|---|
| Dispersion (I) (Magenta) | Used | 1.90 | 0.18 |
| | None | 0.03 | 0.03 |
| Dispersion (II) (Yellow) | Used | 1.67 | 0.22 |
| | None | 0.03 | 0.03 |
| Dispersion (III) (Cyan) | Used | 2.03 | 0.37 |
| | None | 0.20 | 0.05 |

From the above results, it can be seen that the base precursors of this invention provide an image with high maximum density.

Example 4

The following example illustrates the use of an organic silver salt oxidizing agent.

Preparation of benzotriazole silver salt emulsion:

In 3,000 ml of water were dissolved 28 g of gelatin and 13.2 g of benzotriazole and the solution was stirred at 40°C. To the solution was added a solution of 17 g of silver nitrate in 100 ml of water over a period of two minutes.

The pH of the benzotriazole silver salt emulsion was controlled to precipitate excessive salts, which were then removed. Thereafter, the pH of the emulsion was adjusted to 6.0 to provide 400 g of a benzotriazole silver salt emulsion.

A light-sensitive material was prepared as follows using the benzotriazole silver salt emulsion.

| | | |
|---|---|---|
| (a) | Silver Iodobromide Emulsion (as described in Example 1) | 20 g |
| (b) | Benzotriazole Silver Salt Emulsion | 10 g |
| (c) | Dispersion of Dye-Releasing Material (1) (as described in Example 1) | 33 g |
| (d) | 5% Aqueous Solution of the Following Compound | 10 ml |

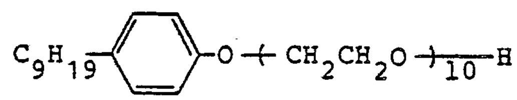

| | | |
|---|---|---|
| (e) | 10% Aqueous Solution of the Following Compound | 4 ml |

$H_2NSO_2N(CH_3)_2$

| | | |
|---|---|---|
| (f) | Solution of 3 g of Base Precursor (1) of this invention in 30 ml of 50:50 vol % Water-Methanol Mixture | |

The foregoing components (a) to (f) were mixed and by following the same procedure as described in Example 1 using the mixture, a light-sensitive sample was prepared. The sample was processed as

described in Example 1. The results obtained are shown below together with results of a comparison sample prepared using the same method as above but without using the base precursor.

| Sample | Maximum Density | Minimum Density |
|---|---|---|
| Sample of This Invention (containing Base Precursor (1) of this invention) | 2.11 | 0.20 |
| Comparison Sample (without any base precursor) | 0.03 | 0.03 |

From the above results, it can be seen that the base precursor of this invention provides images with high density.

**Claims**

1. A heat developable color photographic material comprising a support having thereon at least a photosensitive silver halide, a binder, a dye-releasing material which is reductive to the photosensitive silver halide and releases a hydrophilic mobile dye upon reaction with the photosensitive silver halide by heating, and a base precursor, characterised in that the base precursor is represented by the general formula I

$$[R \leftarrow SO_2CH_2CO_2H)_x]_z \cdot B_y \qquad (I)$$

wherein

R represents an alkyl group, an alkylene group, an aryl group, an arylene group, a monovalent or divalent heterocyclic group, each of which may be unsubstituted or substituted;

B represents a monoacidic or diacidic base having a pKa of not lower than 9 and containing 12 carbon atoms or less;

x is 1 when R represents a monovalent group or 2 when R represents a divalent group;

y is the same as x when B represents a monoacidic base or 1 when B represents a diacidic base; and

z is 2 when R represents a monovalent group and B represents a diacidic base or otherwise 1.

2. The color photographic material of claim 1, characterized in that R is an aryl group, an arylene group, or a heterocyclic group, each of which group may contain one or more substituents.

3. The color photographic material of claim 1, characterized in that R is an aryl group or a heterocyclic group, each of which is substituted with an electron attracting group having a Hammet sigma value of above 0.

4. The color photographic material of claim 3, characterized in that said electron attracting group is a halogen atom, a cyano group, a nitro group, a carbamoyl group or a sulfamoyl group.

5. The color photographic material of claim 1, characterized in that said B is a base having a low volatility and a pKa value of not lower than 10 with a boiling point of not lower than about 150°C.

6. The color photographic material of claim 1, characterized in that said B is a guanidine, a cyclic guanidine, an amidine, a cyclic amidine or a tetraalkylammonium hydroxide.

7. The color photographic material of claim 1, characterized in that said dye-releasing material is a dye-releasing redox compound releasing a hydrophilic diffusible dye and having the formula

$$R_a—SO_2—D$$

wherein

$R_a$ represents a reducing group capable of being oxidized by silver halide and

D represents an image forming dye moiety containing a hydrophilic group.

8. The color photographic material of claim 1, characterized in that said material additionally includes an auxiliary developing agent.

9. The color photographic material of claim 1, characterized in that said material additionally contains an organic silver salt oxidizing agent.

10. The color photographic material of claim 1, characterized in that said material additionally contains a dye-releasing activator.

**Patentansprüche**

1. Wärmeentwickelbares farbphotographisches Material, umfassend einen Träger und darauf befindlich mindestens ein lichtempfindliches Silberhalogenid, ein Bindemittel, ein Farbstoff freisetzendes Material, das gegenüber dem lichtempfindlichen Silberhalogenid reduzierend ist und bei der Reaktion mit dem lichtempfindlichen Silberhalogenid durch Erwärmen einen hydrophilen, mobilen Farbstoff freisetzt, und einen Basenvorläufer, dadurch gekennzeichnet, daß der Basenvorläufer der allgemeinen Formel (I) entspricht

$$(R \text{+} SO_2CH_2CO_2H)_x)_z \cdot B_y \qquad (I)$$

worin bedeuten:

R eine Alkylgruppe, Alkylengruppe, Arylgruppe, Arylengruppe, eine einwertige oder zweiwertige heterocyclische Gruppe, von denen jede unsubstituiert oder substituiert sein kann;

B eine einsäurige oder zweisäurige Base mit einem pKa von nicht weniger als 9, die 12 oder weniger Kohlenstoffatome enthält;

x = 1, wenn R eine einwertige Gruppe bedeutet oder 2, wenn R eine zweiwertige Gruppe bedeutet;

y gleich wie x, wenn B eine einsäurige Base bedeutet oder 1, wenn B eine zweisäurige Base bedeutet; und

z = 2, wenn R eine einwertige Gruppe und B eine zweisäurige Base bedeutet oder anderenfalls 1.

2. Farbphotographisches Material nach Anspruch 1, dadurch gekennzeichnet, daß R eine Arylgruppe, Arylengruppe oder eine heterocyclische Gruppe bedeutet, wobei jede dieser Gruppen einen oder mehrere Substituenten enthalten kann.

3. Farbphotographisches Material nach Anspruch 1, dadurch gekennzeichnet, daß R eine Arylgruppe oder heterocyclische Gruppe bedeutet, die jeweils mit einer Elektronen anziehenden Gruppe mit einem Hammet-Sigma-Wert von über 0 substituiert ist.

4. Farbphotographisches Material nach Anspruch 2, dadurch gekennzeichnet, daß die Elektronen anziehende Gruppe ein Halogenatom, eine Cyanogruppe, Nitrogruppe, Carbamoylgruppe oder Sulfamoylgruppe ist.

5. Farbphotographisches Material nach Anspruch 1, dadurch gekennzeichnet, daß B eine Base mit geringer Flüchtigkeit und einem pKa-Wert von nicht weniger als 10 sowie einem Siedepunkt von nicht weniger als etwa 150°C ist.

6. Farbphotographisches Material nach Anspruch 1, dadurch gekennzeichnet, daß B ein Guanidin, zyklisches Guanidin, Amidin, zyklisches Amidin oder ein Tetraalkylammoniumhydroxid ist.

7. Farbphotographisches Material nach Anspruch 1, dadurch gekennzeichnet, daß das Farbstoff freisetzende Material eine Farbstoff freisetzende Redoxverbindung ist, die einen hydrophilen, diffusionsfähigen Farbstoff freisetzt und der Formel

$$Ra—SO_2—D$$

entspricht, worin bedeuten:

Ra eine reduzierende Gruppe, die in der Lage ist, durch Silberhalogenid oxidiert zu werden und

D einen bilderzeugenden Farbstoffteil, der eine hydrophile Gruppe enthält.

8. Farbphotographisches Material nach Anspruch 1, dadurch gekennzeichnet, daß das Material zusätzlich ein Hilfsentwicklungsmittel enthält.

9. Farbphotographisches Material nach Anspruch 1, dadurch gekennzeichnet, daß das Material zusätzlich ein organisches Silbersalz-Oxidationsmittel enthält.

10. Farbphotographisches Material nach Anspruch 1, dadurch gekennzeichnet, daß das Material zusätzlich einen Farbstoffreisetzungsaktivator enthält.

**Revendications**

1. Matériau pour photographie en couleur développable à chaud comprenant un support portant au moins un halogénure d'argent photosensible, un liant, un matériau libérant un colorant qui est réducteur vis-à-vis de l'halogénure d'argent photosensible et libère un colorant hydrophile mobile par réaction avec l'halogénure d'argent photosensible au chauffage, et un précurseur de base, caractérisé en ce que le précurseur de base est représenté par la formule générale (I)

$$(R \text{+} SO_2CH_2CO_2H)_x)_z \cdot B_y \qquad (I)$$

dans laquelle

R représente un groupe alkyle, un groupe alkylène, un groupe aryle, un groupe arylène, un groupe hétérocyclique monovalent ou divalent, chacun de ces groupes pouvant être insubstitué ou substitué;

B représente une base monoacide ou diacide ayant un pKa non inférieur à 9 et contenant au plus 12 atomes de carbone;

X est 1 quand R représente un groupe monovalent ou 2 quand R représente un groupe divalent;

y est identique à x quand B représente une base monoacide ou 1 quand B représente une base diacide; et

z est 2 quand R représente un groupe monovalent et B représente une base diacide, ou 1 sinon.

2. Matériau pour photographie en couleur selon la revendication 1, caractérisé en ce que R est un groupe aryle, un groupe arylène ou un groupe hétérocyclique, chacun de ces groupes pouvant contenir un ou plusieurs substituants.

3. Matériau pour photographie en couleur selon la revendication 1, caractérisé en ce que R est un groupe aryle ou un groupe hétérocyclique, chacun de ces groupes est substitué par un groupe attracteur d'électron ayant une valeur de Hammet sigma supérieure à 0.

4. Matériau pour photographie en couleur selon la revendication 3, caractérisé en ce que le groupe attracteur d'électron est un atome d'halogène, un groupe cyano, un groupe nitro, un groupe carbamyle ou un groupe sulfamyle.

5. Matériau pour photographie en couleur selon la revendication 1, caractérisé en ce que B est une base ayant une faible volatilité et une valeur de pKa non inférieure à 10 avec un point d'ébullition non inférieur à 150°C.

6. Matériau pour photographie en couleur selon la revendication 1, caractérisé en ce que B est une guanidine, une guanidine cyclique, une amidine, une amidine cyclique ou un hydroxyde de tétraalkyl ammonium.

7. Matériau pour photographie en couleur selon la revendication 1, caractérisé en ce que ledit matériau libérant un colorant est un composé redox libérant un colorant, libérant un colorant hydrophile diffusible et ayant la formule

$$R_a—SO_2—D$$

dans laquelle

$R_a$ représente un groupe réducteur capable d'être oxydé par un halogénure d'argent et

D représente une partie colorant formateur d'image contenant un groupe hydrophile.

8. Matériau pour photographie en couleur selon la revendication 1, caractérisé en ce que ledit matériau contient en outre un agent développateur auxiliaire.

9. Matériau pour photographie en couleur selon la revendication 1, caractérisé en ce que ledit matériau contient en outre un sel d'argent organique oxydant.

10. Matériau pour photographie en couleur selon la revendication 1, caractérisé en ce que ledit matériau contient en outre un activateur de libération du colorant.